(19) Europäisches Patentamt

European Patent Office

Office européen des brevets

(11) **EP 0 648 110 B1**

(12) **EUROPÄISCHE PATENTSCHRIFT**

(45) Veröffentlichungstag und Bekanntmachung des Hinweises auf die Patenterteilung:
**18.09.1996 Patentblatt 1996/38**

(21) Anmeldenummer: 93915766.5

(22) Anmeldetag: 01.07.1993

(51) Int. Cl.[6]: **A61K 7/16**

(86) Internationale Anmeldenummer:
**PCT/EP93/01701**

(87) Internationale Veröffentlichungsnummer:
**WO 94/01080 (20.01.1994 Gazette 1994/03)**

(54) **FLÜSSIGE ZAHNREINIGUNGSMITTEL**

LIQUID TOOTH-CLEANING AGENTS

DENTIFRICE LIQUIDE

(84) Benannte Vertragsstaaten:
**AT BE CH DE DK ES FR GB GR IT LI NL PT SE**

(30) Priorität: **10.07.1992 DE 4222739**

(43) Veröffentlichungstag der Anmeldung:
**19.04.1995 Patentblatt 1995/16**

(60) Teilanmeldung: **95111471.9**

(73) Patentinhaber: **Henkel Kommanditgesellschaft auf Aktien**
**40191 Düsseldorf (DE)**

(72) Erfinder:
• SCHUMANN, Klaus
  **D-40699 Erkrath (DE)**
• FÖRG, Franz
  **D-40764 Langenfeld (DE)**
• GUTZSCHEBAUCH, Claus
  **D-41470 Neuss (DE)**

(56) Entgegenhaltungen:
EP-A- 0 216 189          EP-A- 0 275 706
WO-A-87/05501           WO-A-88/00463
GB-A- 2 082 062          GB-A- 2 100 983
GB-A- 2 149 661          US-A- 4 401 648
US-A- 4 623 536          US-A- 4 837 008
US-A- 5 073 363

**Beschreibung**

Gegenstand der Erfindung sind flüssige, ihre Fließfähigkeit langfristig beibehaltende Zahnreinigungsmittel.

Zahnpasten zeigen üblicherweise pseudoplastisches Verhalten und lassen sich durch die Angabe einer Fließgrenze charakterisieren. Sie lassen sich aus einer Tube ausdrücken und zeigen einen stabilen Stand auf der Zahnbürste. Diese Anwendungsform erfordert vom Verbraucher jedoch einige Sorgfalt beim Dosieren, da der Zahnpastastrang plötzlich aus der Tube austreten kann. Oftmals trennt sich dann ein Teil des Zahnpastastranges entweder schon beim Aufbringen auf die Zahnbürste oder beim Einführen der Zahnbürste in den Mund ab und verbleibt ungenutzt im Mundraum oder im Waschbecken.

Die vorliegende Erfindung hat zur Aufgabe, ein flüssiges, die Fließfähigkeit langfristig beibehaltendes Zahnreinigungsmittel bereitzustellen. Langfristige Fließfähigkeit im Sinne der Erfindung bedeutet, daß das Zahnreinigungsmittel in flüssiger Form vorliegt und je nach eingestellter Viskosität dünnflüssig aus einem Vorratsbehälter herausläuft oder in Tropfenform aus einem Behälter entnommen werden kann und daß die einmal eingestellte Konsistenz über einen längeren Zeitraum bis zum Verbrauch beibehalten wird. Das Zahnreinigungsmittel soll bereits beim Auftragen auf die Zahnbürste zwischen die Borstenköpfe eindringen und nicht erst nach einigen Bürstenstrichen, wie es bei einer herkömmlichen Zahnpasta der Fall ist. Nach dem Aufbringen soll das flüssige Zahnreinigungsmittel bis zur Anwendung auf und zwischen den Borsten verbleiben und nicht den Borstenschaft herunterlaufen. Ein solches flüssiges Zahnreinigungsmittel ermöglicht eine genaue Dosierung und einen sehr sparsamen Verbrauch. Eine direkte Berührung des Vorratsbehälters mit der Bürste ist im Gegensatz zur Zahnpasta, die aus einer Tube ausgedrückt wird, nicht notwendig. Dies ermöglicht eine besonders hygienische Anwendung. Weiterhin ermöglicht ein flüssiges Zahnreinigungsmittel eine vollständige Entleerung des Vorratsbehälters, was bei einer Zahnpastatube kaum möglich ist.

Die Formulierung eines Zahnreinigungsmittels in flüssiger Form darf selbstverständlich die eigentliche zahnreinigende und zahnpflegende Wirkung des Mittels nicht beeinträchtigen.

Aus US-A-4,623,536 sind Zahnpasten mit wenigstens 60 Gew.-% Natriumbicarbonat bekannt, die als Verdickungsmittel Xanthan-Gum enthalten können. In US-A-4,837,008 sind wasserfreie Zahnpasten- oder -Gele beschrieben, die ein Peroxid und ein Bicarbonat-Salz und zur Verdickung Xanthan-Gum enthalten können.

Aus der deutschen Auslegeschrift DE-16 67 871 ist bekannt, in flüssigen Zahnpflegemitteln das Hydrokolloid Xanthan als Suspendier- und Verdickungsmittel zu verwenden.

Es zeigte sich jedoch, daß die gestellte Anforderung der langfristigen Fließfähigkeit nicht mehr erfüllt ist, wenn Xanthan in Gegenwart von Kieselsäureabrasiva verwendet wird. So nimmt die Fließfähigkeit eines solchen Flüssigzahnpflegemittels nach kurzer Zeit aufgrund der Ausbildung überhöhter Fließgrenzen sowie dynamischer Viskositäten ab.

Es wurde nun überraschenderweise gefunden, daß die Fließfähigkeit eines flüssigen Zahnreinigungsmittels langfristig erhalten bleibt, wenn man als Konsistenzregler Xanthan in Verbindung mit ganz bestimmten viskositätsstabilisierenden Substanzen verwendet. Unter diesen viskositätsstabilisierenden Substanzen sind im Sinne der Erfindung solche Stoffe zu verstehen, die ein allmähliches "Nachdicken" des Flüssigzahnreinigungsmittels, bzw. die Ausbildung einer erhöhten Fließgrenze, verhindern.

Diese Substanzen im Sinne der Erfindung sind stickstoffhaltige kationische oder ampholytische Tenside.

Gegenstand der Erfindung sind deshalb Wasser und Feuchthaltemittel enthaltende Zahnreinigungsmittel in Form einer flüssigen Zubereitung mit langfristiger Fließfähigkeit und einer Viskosität von 2 000 bis 10 000, vorzugsweise 5 000 bis 8 000 mPa · s bei 25 °C, wobei als Konsistenzregler Xanthan in einer Menge von 0,02 bis 3,0 Gew.-% und als viskositätsstabilisierender Zusatz ein stickstoffhaltiges kationisches oder ampholytisches Tensid in einer Menge von 0,01 bis 5,0 Gew.-% enthalten ist.

Weiterhin eignen sich als viskositätsstabilisierende Zusätze:

a) hydroxypropylsubstituierte Hydrokolloide
b) N-Acyl-Sarkosinate
c) N-Acyl-Tauride oder
d) Polyethylenglykol/Polypropylenglykol-Copolymere mit einem mittleren Molekulargewicht von 1 000 bis 5 000.

Gemäß einer weiteren Ausführung der Erfindung ist daher als viskositätsstabilisierender Zusatz eine Kombination des stickstoffhaltigen kationischen oder amphoteren Tensids mit einer Verbindung aus der Gruppe der unter a) bis d) aufgeführten Verbindungen in einer Menge von 0,01 bis 10 Gew.-% enthalten, wobei sich alle Mengenangaben auf das gesamte Zahnreinigungsmittel beziehen.

Xanthan ist vorzugsweise in einer Menge von 0,01 bis 0,8 Gew.-%, bezogen auf das gesamte Zahnreinigungsmittel, enthalten.

Hydroxypropylsubstituierte Hydrokolloide sind vorzugsweise in einer Menge von 0,01 bis 0,5 Gew.-%, bezogen auf das gesamte Zahnreinigungsmittel, enthalten; bevorzugte Vertreter sind Hydroxypropylguar, Hydroxypropylcellulose, Alginsäurepropylenglykolester oder Carboxyethyl- und Carboxymethylcellulosepropylenglykolester. Die Propylenglykol-Funktion kann demnach sowohl über eine Ether- als auch über eine Estergruppierung gebunden sein; der Veretherungs- bez. Veresterungsgrad pro Hydrokolloid-Monomer-Einheit liegt dabei in dem üblichen Bereich von 0,25 bis ca. 1.

Besonders bevorzugt sind Hydroxypropylguar und Alginsäurepropylenglykolester, die in einer Menge von 0,01 bis 0,5 Gew.-%, bezogen auf das gesamte Zahnreinigungsmittel, enthalten sind.

Das Gewichtsverhältnis Xanthan/hydroxypropylsubstituiertes Hydrokolloid beträgt dabei 20 : 1 bis 2 : 1, vorzugsweise 9 : 1 bis 3 : 1.

Kationische stickstoffhaltige Tenside sind z. B. quartäre Ammoniumverbindungen, z. B. Cetyltrimethylammoniumchlorid, Dicetyldimethylammoniumchlorid oder Distearyldimethylammoniumchlorid.

Ampholytische Tenside sind z. B. Betaine der allgemeinen Formel

$$R^1 \overset{+}{N}(CH_3)_2CH_2COO^-,$$

wobei $R^1$ z. B. Lauryl, Myristyl, Cetyl, Stearyl, Kokosalkyl, Talgalkyl oder hydriertes Talgalkyl darstellt, Betaine der allgemeinen Formel

$$R^2C(O)NH(CH_2)_n\overset{+}{N}(CH_3)_2CH_2COO^-,$$

wobei $R^2$ z. B. alle für $R^1$ genannten Bedeutungen haben kann und n eine ganze Zahl von 1 bis 6 ist, z. B. Lauramidopropyldimethylbetain oder Cocoamidopropyldimethylbetain.

Vorzugsweise sind die Betaine in einer Menge von 0,01 bis 1,5 Gew.-%, bezogen auf das gesamte Zahnreinigungsmittel, enthalten. Besonders bevorzugt sind Lauramido- und Cocoamidopropylbetain. N-Acyl-Sarkosinate und N-Acyl-Tauride sind weitere viskositätsstabilisierende Substanzen im Sinne der Erfindung.

In den N-Acyl-Sarkosinaten der allgemeinen Formel

$$R^3C(O)N(CH_3)CH_2COO^-$$

und den N-Acyl-Tauriden der allgemeinen Formel

$$R^4C(O)N(CH_3)CH_2CH_2SO_3^-,$$

können $R^3$ und $R^4$ z. B. die für $R^1$ genannten Bedeutungen haben.

Als Polyethylenglykol/Propylenglykol-Copolymere mit einem mittleren Molekulargewicht von 1 000 bis 5 000 können Block-Polymere oder statistische Copolymere eingesetzt werden. Vorzugsweise enthalten die Zahnreinigungsmittel statistische Copolymere, die in einer Menge von 0,02 bis 5,0 Gew.-%, bezogen auf das gesamte Zahnreinigungsmittel, enthalten sind.

Die Mischung aus Xanthan und dem viskositätsstabilisierenden Zusatz ist in einen flüssigen Träger eingearbeitet. Der flüssige Träger besteht aus Wasser und der Feuchthaltung dienenden Substanzen, beispielsweise Polyalkoholen, wie z. B. Glycerin, Sorbit, Xylit, 1,2-Propylenglykol oder Polyethylenglykolen mit einem mittleren Molekulargewicht von 200 bis 1 500. Diese Feuchthaltemittel sind in einer Menge von 15 bis 85 Gew.-%, bezogen auf das gesamte Zahnreiniungsmittel, enthalten.

Weitere Bestandteile der erfindungsgemäßen Zahnreinigungsmittel sind:

- Putzkörper, wie z. B. Fällungs- und Gelkieselsäuren, die selbst keine nennenswerten Beitrag zum Verdicken leisten, Calciumphosphate, Polymetaphosphate, Aluminiumoxid und Aluminiumhydroxid, wobei die Putzkörper üblicherweise in einer Menge von 10 bis 25 Gew.-%, bezogen auf das gesamte Zahnreinigungsmittel, enthalten sind,

- Lösungsvermittler, wie z. B. PEG-30-Glycerinmonostearinsäureester, PEG-60-Glycerintri(hydroxystearinsäure)ester

- Tenside, z. B. Aniontenside, Amphotenside, nichtionische Tenside, vorzugsweise Natriumlaurylsulfat, Natriumfettalkoholethersulfate, Fettsäureglycerinestersulfat, Fettalkohol-EO-ether, Fettalkohololigosaccharide,

- wasserlösliche Fluorkomponenten, z. B. Fluoride und Monofluorphosphate,

- Antizahnsteinwirkstoffe, z. B. Phosphonate, bevorzugt Azacycloheptan-2,2-diphosphonat, Pyrophosphate (Dinatriumsalz oder Tetrakaliumsalz), Polyphosphate (Tri- und Tetrapolyphosphat),

- pH-Stellmittel, z. B. Mono-, Di-, Trialkalimonophosphate, Citrate,

- Süßungsmittel, z. B. Saccharin, Acesulfam-K, Cyclamat, Aspartam,

- Aromaöle und Pflanzenextrakte, z. B. Pfefferminzöl, Krauseminzöl, Eukalyptusöl, Anisöl, Fenchelöl, Kümmelöl, Menthylacetat, Zimtaldehyd, Vanillin und Thymol sowie Aromastoffe mit Erdbeer-, Himbeer-, Ananas-, Apfel- oder Orangegeschmack,

- Mikrobiostatika

- sowie Ethanol und Farbstoffe.

Die erfindungsgemäßen flüssigen Zahnreinigungsmittel besitzen bei 25 °C eine Viskosität von etwa 2 000 bis 10 000, vorzugsweise 5 000 bis 8 000 mPas; sie behalten ihre Fließfähigkeit auch in Gegenwart von Kieselsäureabrasiva und in Verbindung mit einem hohen Gehalt an Feuchthaltemitteln langfristig bei. Sie können aus einem Vorratsbehälter durch Überkopfstellen bequem in Tropfenform entnommen werden.

Die erfindungsgemäßen Zahnreinigungsmittel werden in einer Menge von ca. 1 g auf die Borsten einer Zahnbürste flüssig aufgetragen, um in herkömmlicher Weise als Zahnputzmittel zur Anwendung zu kommen. Die erfindungsgemäßen Zahnreinigungsmittel können desweiteren als Mundwasser zum Spülen des Mund- und Rachenraumes sowie der Zahnzwischenräume verwendet werden. Zur Bereitung eines Mundwassers gibt man ca. 1 g des erfindungsgemäßen Zahnreinigungsmittels auf 20 ml Wasser.

Die folgenden Beispiele sollen den Erfindungsgegenstand näher erläutern, ohne ihn jedoch hierauf zu beschränken.

**Beispiel**

Es wurden flüssige Zahnreinigungsmittel der folgenden Zusammensetzungen hergestellt.

Die Zahnreinigungsmittel wurden in verschlossenen 250 ml-Schraubdeckelgläsern eine Stunde lang auf 25°C temperiert, dann wurde die Viskosität bestimmt. Die Viskositätsmessungen wurden mit einem Brookfield Synchro-lectric-Viscosimeter bei 25°C (Typ RVF, Spindel 3) durchgeführt; die Drehzahl betrug 4 Umdrehungen pro Minute.

## 1. Flüssiges Zahnreinigungsmittel, transparent

| | |
|---|---|
| Natriumlaurylsulfat | 1,50 % |
| PEG-30-Glycerinmonostearinsäureester | 0,5 % |
| Cocoamidopropylbetain | 0,18 % |
| Dinatriumphosphat | 0,2 % |
| Wasser | 24,668 % |
| Aromaöl | 1,1 % |
| Natriumhydroxid | 0,315 % |
| Ethanol (96 Vol.-%) | 5,0 % |
| Glycerin (86 %) | 28,38 % |
| Polyethylenglykol (MG 400) | 3,0 % |
| Fällungskieselsäure (Sident 12 DS) | 12,0 % |
| Farbstoff | 0,001 % |
| Xanthan | 0,2 % |
| Natriumfluorid | 0,231 % |
| Sorbit (70 %) | 21,7 % |
| Saccharin (Natriumsalz) | 0,2 % |
| Azacycloheptan-2,2-diphosphonat (Natriumsalz) | 1,0 % |
| Viskosität bei 25°C: | 9.000 mPa · s |

Zur Verwendung als Zahnputzmittel werden ca. 1 g des Zahnreinigungsmittels auf die Borsten einer Zahnbürste flüssig aufgetragen und in herkömmlicher Weise zum Zähneputzen benutzt.

Zur Verwendung als Mundwasser werden ca. 1 g des Zahnreinigungsmittels in 20 ml Wasser dispergiert.

## Patentansprüche

1. Wasser- und Feuchthaltemittel enthaltende Zahnreinigungsmittel in Form einer flüssigen Zubereitung mit langfristiger Fließfähigkeit und einer Viskosität von 2 000 bis 10 000, vorzugsweise 5 000 bis 8 000 mPa · s bei 25 °C, wobei als Konsistenzregler Xanthan in einer Menge von 0,02 bis 3,0 Gew.-%, und als viskositätsstabilisierender Zusatz ein stickstoffhaltiges kationisches oder ampholytisches Tensid in einer Menge von 0,01 bis 5,0 Gew.-% enthalten ist, wobei sich alle Mengenangaben auf das gesamte Zahnreinigungsmittel beziehen.

2. Zahnreinigungsmittel nach Anspruch 1,
   dadurch gekennzeichnet,
   daß als stickstoffhaltiges Tensid ein Betain in einer Menge von 0,01 bis 1,5 Gew.-% enthalten ist.

3. Zahnreinigungsmittel nach Anspruch 2,
   dadurch gekennzeichnet,
   daß das Betain Lauramido- oder Cocoamidopropyldimethylbetain ist.

4. Zahnreinigungsmittel nach Anspruch 1 bis 3,
   dadurch gekennzeichnet,
   daß Xanthan in einer Menge von 0,1 bis 0,8 Gew.-%, enthalten ist.

5. Zahnreinigungsmittel nach Anspruch 1, dadurch gekennzeichnet, daß als viskositätsstabilisierender Zusatz eine Kombination des stickstoffhaltigen kationischen oder ampholytischen Tensids mit einer Verbindung aus der Gruppe

a) der hydroxypropylsubstituierten Hydrocolloide

b) der N-Acyl-Sarkoside

c) der N-Acyl-Tauride oder

d) der Polyethylenglycol/Polypropylenglycol-Copolymere mit einem mittleren Molekulargewicht von 1 000 bis 5 000

in einer Menge von 0,1 bis 10 Gew.-% enthalten ist.

6. Zahnreinigungsmittel nach Anspruch 5,
dadurch gekennzeichnet,
daß ein hydroxypropylsubstituiertes Hydrokolloid in einer Menge von 0,01 bis 0,5 Gew.-% enthalten ist und ausgewählt ist aus der Gruppe Carboxyethyl- oder Carboxymethylcellulosepropylenglykolester, Alginsäurepropylenglykolester, Hydroxypropylcellulose oder Hydroxypropylguar.

7. Zahnreinigungsmittel nach Anspruch 5 oder 6,
dadurch gekennzeichnet,
daß ein hydroxypropylsubstituierte Hydrokolloid in einer Menge von 0,01 bis 0,5 Gew.-% enthalten ist und ausgewählt ist aus Hydroxypropylguar oder Alginsäurepropylenglykolester.

## Claims

1. Water- and humectant-containing tooth-cleaning formulations in the form of a liquid preparation with long-term fluidity and a viscosity of 2,000 to 10,000 and preferably 5,000 to 8,000 mPa·s at 25°C, xanthan in a quantity of 0.02 to 3.0% by weight being present as consistency regulator and a nitrogen-containing cationic or ampholytic surfactant in a quantity of 0.01 to 5.0% by weight being present as viscosity stabilizer (all the percentages by weight mentioned being based on the tooth-cleaning formulation as a whole).

2. Tooth-cleaning formulations as claimed in claim 1, characterized in that a betaine in a quantity of 0.01 to 1.5% by weight is present as the nitrogen-containing surfactant.

3. Tooth-cleaning formulations as claimed in claim 2, characterized in that the betaine is lauramido- or cocoamidopropyl dimethyl betaine.

4. Tooth-cleaning formulations as claimed in claims 1 to 3, characterized in that xanthan is present in a quantity of 0.1 to 0.8% by weight.

5. Tooth-cleaning formulations as claimed in claim 1, characterized in that a combination of the nitrogen-containing cationic or ampholytic surfactant with a compound from the group consisting of

a) hydroxypropyl-substituted hydrocolloids

b) N-acyl sarcosides

c) N-acyl taurides or

d) polyethylene glycol/polypropylene glycol copolymers with an average molecular weight of 1,000 to 5,000

is present as the viscosity stabilizer in a quantity of 0.1 to 10% by weight.

6. Tooth-cleaning formulations as claimed in claim 5, characterized in that a hydroxypropyl-substituted hydrocolloid is present in a quantity of 0.01 to 0.5% by weight and is selected from the group consisting of carboxyethyl or carboxymethyl cellulose propylene glycol ester, alginic acid propylene glycol ester, hydroxypropyl cellulose or hydroxypropyl guar.

7. Tooth-cleaning formulations as claimed in claim 5 or 6, characterized in that a hydroxypropyl-substituted hydrocolloid is present in a quantity of 0.01 to 0.5% by weight and is selected from hydroxypropyl guar or alginic acid propylene glycol ester.

## Revendications

1. Dentifrice contenant de l'eau et des agents d'humidification, sous forme d'une préparation liquide ayant une aptitude à l'écoulement de longue durée et une viscosité allant de 2 000 à 10 000, de préférence de 5 000 à 8 000 mPa.s à 25°C, dans lequel comme régulateur de la consistance on inclut du xanthanne en quantité allant de 0,02 à 3,0 % en poids et comme additif stabilisant la viscosité un agent tensioactif cationique ou ampholytique contenant de l'azote, en quantité allant de 0,01 à 5,0 % en poids, dans lesquels toutes les données en poids se rapportant au dentifrice total.

2. Dentifrice selon la revendication 1, caractérisé en ce que comme agent tensioactif contenant de l'azote, une bétaïne est contenue, en quantité allant de 0,01 à 1,5 % en poids.

3. Dentifrice selon la revendication 2, caractérisé en ce que la bétaïne est la lauramido- ou la cocoamidopropyldiméthylbétaïne.

4. Dentifrice selon la revendication 1 à 3, caractérisé en ce que le xanthanne est inclus en quantité allant de 0,1 à 0,8 % en poids.

5. Dentifrice selon la revendication 1, caractérisé en ce que comme additif qui stabilise la viscosité, une combinaison de l'agent tensioactif, cationique ou ampholytique renfermant de l'azote avec un composé du groupe :

   a) des hydrocolloïdes substitués par un hydroxypropyle,
   b) des N-acyl-sarcosides,
   c) des N-acyl-taurides ou
   d) des copolymères polyéthylèneglycol/polypropylèneglycol ayant un poids moléculaire moyen de 1 000 à 5 000,

   est contenue en quantité allant de 0,1 à 10 % en poids.

6. Dentifrice selon la revendication 5, caractérisé en ce qu'un hydrocolloïde substitué par un hydroxypropyle est contenu en quantité allant de 0,01 à 0,5 % en poids et est choisi dans le groupe des esters de propylèneglycol de carboxyéthyl- et de carboxyméthylcellulose, des esters de propylèneglycol d'acide alginique, de l'hydroxypropylcellulose et de l'hydroxypropylguar.

7. Dentifrice selon la revendication 5 ou la revendication 6, caractérisé en ce qu'un hydrocolloïde substitué par un hydroxypropyl est contenu en quantité allant de 0,01 à 0,5 % en poids et est choisi parmi l'hydroxypropylguar et l'ester de propylèneglycol d'acide alginique.